# EUROPEAN PATENT SPECIFICATION

(11) **EP 3 092 487 B1**
(45) Date of publication and mention of the grant of the patent: **12.04.2023**
(21) Application number: 15710288.0
(22) Date of filing: 10.01.2015
(51) Int. Cl.: G01N 33/03, G01N 21/51, G01N 21/93

(54) **A METHOD FOR DETERMINATION OF CONTENT OF HYDROPHOBIC COMPOUNDS IN WATER-MISCIBLE ORGANIC LIQUIDS**
VERFAHREN ZUR BESTIMMUNG DES INHALTS VON HYDROPHOBEN VERBINDUNGEN IN WASSERMISCHBAREN ORGANISCHEN FLÜSSIGKEITEN
PROCÉDÉ PERMETTANT DE DÉTERMINER LA TENEUR EN COMPOSÉS HYDROPHOBES DANS DES LIQUIDES ORGANIQUES HYDROSOLUBLES

(30) Priority: 10.01.2014 SK 500022014; 09.01.2015 SK 500012015
(43) Date of publication of application: 16.11.2016
(73) Proprietor: Ústav Experimentálnej Fyziky Sav, 04001 Kosice (SK); Centrum Vedecko Technickych Informácii Srl., 811 04 Bratislava (SK)
(72) Inventor: SEDLÁK, Marián, 04001 Kosice (SK); RAK, Dmytro, 04023 Kosice (SK)
(74) Representative: Holoubková, Mária
(86) International application number: PCT/SK2015/050002
(87) International publication number: WO 2015/105463

(56) References cited:
- EP-A1- 2 306 177
- EP-A2- 0 361 904
- US-A- 5 736 407
- US-A1- 2008 125 554

## Description

### Technical Field

The invention concerns a method for determination of content of hydrophobic compounds in water-miscible organic liquids by utilization of nanosegregation in aqueous solutions.

### Current State of the Art

Water-miscible organic liquids represent a group of organic compounds which are widely used in the food processing and industry. Among these organic liquids, alcohols play an important role. For example, methanol (methyl alcohol) is used as a solvent and starting material for the synthesis of organic compounds. Ethanol has a wide application in medicine, cosmetics and pharmaceutical industry; it is used in the manufacture of alcoholic beverages. Other alcohols such as isopropanol (isopropyl alcohol), and tert-butanol (tert-butyl alcohol) are components of the cleaning agents, are used as solvents, etc. Acetone has a wide use in industry; it is used in large amounts in the synthesis of methyl methacrylate (organic glass) and as a solvent in other organic synthesis (including synthesis processes of pharmaceutical chemistry). Organic ethers, cyclic and acyclic, are used in industry as solvents for polymers and aprotic reaction media for certain organic synthesis (reactions that involve organometallics). Especially important are tetrahydrofuran (THF) and dimethoxyethane (glyme, DME). Dimethoxyethane is used among other things as a component of lithium ion battery electrolyte. Acetone, tetrahydrofuran, isopropyl alcohol and some other organic liquids are also widely used as solvents for varnishes and paints.

Important factor with respect to the area of alcohols use (medicine, pharmaceutical industry, food industry) is their purity. During their production, transportation, and processing, there is a contact with the equipment and consequently contamination of alcohols with higher alkanes (paraffins), which constitute the industrial lubricants, or with phthalates that come from the contact with plastic containers, stoppers, etc. For these reasons, the determination of alkanes in alcohols is an important task.

Nowadays there are quite complicated chromatographic methods for the determination of alkanes in liquid media, which require complex and expensive technical equipment, and qualified personnel. These methods include:
- high performance liquid chromatography (HPLC) [L. R. Snyder, J.J. Kirkland, and J. W. Dolan, Introduction to Modern Liquid Chromatography, John Wiley & Sons, New York, 2009; M. C. McMaster, HPLC: A Practical User's Guide, 2nd ed., Wiley, Hoboken, New Jersey, 2007]
- Gas chromatography (GC) [H.-J. Hubschmann, Handbook of GC/MS: Fundamentals and Applications, Wiley-VCH, Weinheim, Germany, 2001; R.L. Grob, E.F.Barry, Modern Practice of Gas Chromatography, 4th ed., Wiley, Hoboken, New Jersey, 2004; O.D. Sparkman, Z. Penton, F.G. Kitson, Gas Chromatography and Mass Spectrometry: A Practical Guide, 2nd ed., San Diego, Academic Press, 2011].

In conjunction with these chromatographic methods, there are different types of detectors used, such as a mass spectrometer (MS), a flame ionization detector (FID), a catalytic combustion detector (CCD), an infrared detector (IRD), or refractive index detector (RI), etc.

Determination of the content of hydrophobic compounds in lower alcohols and in some other water miscible liquids is especially important with regard to the food quality requirements. Some of the hydrophobic compounds, such as polycyclic aromatic hydrocarbons (PACs), dioxins and polychlorinated biphenyls (PCBs) are listed in the Commission Regulation (EC) No 1881/2006 (separately Commission Regulation (EU) No 835/2011 for PACs) as controlled substances in certain types of foodstuff with maximum abundance that cannot exceed 1 to 35 ppm dependent on the foodstuff [Commission Regulation (EC) No 1881/2006 of 19 December 2006 setting maximum levels for certain contaminants in foodstuffs; Commission Regulation (EU) No 835/2011 of 19 August 2011 amending Regulation (EC) No 1881/2006 as regards maximum levels for polycyclic aromatic hydrocarbons in foodstuffs] .

Others, such as phthalates, alkanes and their derivatives, hydrophobic aromatic compounds, etc. are controlled by the Commission Directive 2002/72/EC as contaminants that can be transferred to foodstuffs from the materials intended for contact with foodstuffs (plastic materials and articles) [Commission Directive 2002/72/EC of 6 August 2002 relating to plastic materials and articles intended to come into contact with foodstuffs]. Monitoring of the abundance of those contaminants in alcoholic beverages, solvents used in the food production (such as glycerol, ethanol, etc.) and extracting solvents used in the production of foodstuffs and food ingredients (such as acetone, ethanol, methanol, isopropanol, etc.) is very important.

The only methods that can be applied for confirmatory purposes in analysis of contaminants mentioned above are gas chromatography based methods, such as GC/MS (Gas Chromatography coupled to Mass Spectrometry), GC/MS/MS (Gas Chromatography coupled to Tandem Mass Spectrometry), etc. Specific requirements for GC/MS methods to be complied with for confirmatory purposes are laid down in Annex I-IV of Commission Regulation (EU) No 589/2014 for certain foodstuffs and in Annex I of Commission Regulation (EC) No 152/2009 of 27 January 2009 for feed. The screening methods may comprise bioanalytical and GC/MS methods [Commission Regulation (EU) No 589/2014 of 2 June 2014 laying down methods of sampling and analysis for the control of levels of dioxins, dioxin-like PCBs and non-dioxin-like PCBs in certain foodstuffs and repealing Regulation (EU) No 252/2012; Commission Regulation (EC) No 152/2009 of 27 January 2009 laying down the methods of sampling and analysis for the official control of feed]. In spite of the fact that GC/MS methods allow analyzing samples with high resolution and low detection limits, they demand highly qualified personnel, time consuming calibrations, and expensive technical equipment. For example, the price of a typical GC/MS instrument that can be used for analysis of xenobiotics spans from 100 to 150 thousands of Euros (and up to 300 thousands of Euros for instrument with Tandem or High Resolution MS). Typical price for one GC/MS measurement fall in the range from 50 to 125 Euros dependent on the type of the substance (i.e. approximately 500 Euros for all mentioned compounds). Moreover, complicated sample preparations such as LLE (Liquid-Liquid Extraction), SPME (Solid Phase Microextraction) or HS-SPME (Head-Space Solid Phase Microextraction) that doubles analysis time is usually unavoidable. Bioanalytical methods also require qualified personnel, complicated sample preparation and well equipped laboratory. Sufficient results may be obtained by using HPLC/MS or UHPLC/MS (High Performance Liquid Chromatography coupled to Mass Spectrometer or Ultra HPLC/MS) methods, however, they also require complicated sample preprocessing [Leo M.L. Nollet, Fidel Toldra, Food Analysis by HPLC, Third Edition, CRC Press, 2012]. All of these methods are not applicable for simultaneous analysis of PACs, PCBs, dioxins, phthalates, etc. (i.e. hydrophobic contaminants) and require few distinct measurements to determine if the given sample should be an object of further study. That is against the nature of screening methods that should allow cost-effective high -throughput measurements on large number of samples, thus increasing the chance to discover new incidents with high exposure and health risks for consumers. Therefore searching for faster, more cost-efficient and reliable methods for determination of hydrophobic contaminants in trace amounts (from 1 ppm to 1 ppb) are an object of intense interest. EP 2 306 177, US 2008/125554, US 5 736 407, EP 0 361 904 disclose various turbidity meters for measuring suspensions in fluids

The aim of this invention is the determination of the content of hydrophobic compounds in organic liquid using a time-saving method featuring an applicability in the field and last but not least its affordability in comparison with existing methods, regarding both the price of the instrument as well as the price of one measurement.

### Basis of the Invention

This invention, represents a method for the determination of the content of hydrophobic compounds in water-miscible organic liquids, characterized in that an organic liquid containing hydrophobic compounds from 0.0005% to 0.1 mass % is mixed with water such that a mixture containing from 3% to 45% of the organic liquid is made at temperatures at which such a mixture is in a liquid phase, which causes segregation of hydrophobic compounds contained in the organic liquid into stable nanoparticles/nanodroplets and subsequently the content of hydrophobic compounds in organic liquid is quantified by means of calibration dependencies of scattering intensity or nanoparticle size or nanoparticles total volume on content of hydrophobic compounds in the organic liquid.

The measurement method according to this invention is based on an unexpected technical effect outside common general knowledge that the hydrophobic compounds contained in organic liquid in given quantities, after being mixed with water under given conditions, neither get molecularly dissolved nor segregate into a macrophase. Instead, they aggregate into discrete, stable nanoparticles with sizes on the order of 100 nm which exhibit such properties and characteristics that in combination lead to the claimed correlation between the concentration of hydrophobic substances and scattering intensity or size or the total volume of all nanoparticles. The sole existence of nanoparticles (any nanoparticles with any properties and characteristics) does not warrant existence of a suitable (usable) correlation. Examples of hydrophobic compounds are alkanes such as decane, hexadecane or octadecane, polychlorinated biphenyls, diethylhexyl phthalate, etc.

Examples of water-miscible organic liquids are alcohols such as ethanol, isopropyl alcohol or tert-butyl alcohol, acetone, dimethoxyethane, etc.

It was found preferable when the content of the organic liquid containing hydrophobic compounds in the mixture with water was from 5 to 25 mass %, more preferably from 10 to 20 mass %.

The content of hydrophobic compounds is quantified by means of calibration curves. Calibration curves are acquired for the same value of the organic liquid content in a mixture with water and the same temperature as sole measurements of samples with unknown content of hydrophobic compounds. Calibration curves are determined using an available method of quantitative analysis for the measurement of scattering intensity from the nanoparticles or the average size or the total volume of nanoparticles in a liquid medium.

The determination of calibration curves and subsequently of the content of hydrophobic compounds in the mixture was realized in the examples presented here mostly by measuring scattering intensity from nanoparticles using static laser light scattering. This method is based on the measurement of light intensity scattered by the nanoparticles comprised of hydrophobic compounds, thus quantifying indirectly their number, size, and density. The intensity of light scattered by nanoparticles is determined simultaneously by the three variables (number concentration, size, and density). In a semiquantitative manner, the intensity is proportional to the number concentration, to the square of density and to the sixth power of the size (radius or diameter) of nanoparticles. The examples in this patent application include also calibration curves obtained by measuring other physical quantities using other methods of quantitative analysis: static and dynamic laser light scattering allowing the measurement of the nanoparticles average size or nanoparticle tracking analysis method allowing measurements of number distributions of nanoparticle sizes and hence also the total volume of all nanoparticles. The presented method of measurement of the content of hydrophobic compounds in organic liquids based on the nanosegregation in aqueous solutions is not limited to the use of either static or dynamic laser light scattering or nanoparticle tracking analysis method. Other methods of quantitative analysis measuring size and/or number concentration and/or density of nanoparticles in liquid medium can be used as well.

The nanosegregation effect can be observed within the range of concentrations of organic liquid in the mixture with water from 1 to 60 mass %, whereas this range may differ slightly for various organic liquids. The range practically usable for the presented method is from 3 to 45 mass %, and preferred from 10 to 20 mass %. The nanosegregation takes place within the temperature range in which the mixture of organic liquid and water is in a liquid state. For various organic liquids and for various contents of organic liquid in the mixture with water, the preferable interval of temperature values differs.

In comparison with the currently used methods, the method according to this invention is cheaper, mainly due to:
- low costs for the fabrication of the measuring device based on static laser light scattering; mainly thanks to the availability of ever cheaper and cheaper semiconductor lasers and detectors;
- its compactness and possibility to use in the field;
- low reagent costs since the reagent is water

Further advantage of the method is the fact that other methods require highly qualified staff for the operation of sophisticated devices of the HPLC and GC-MS (High Performance Liquid Chromatography and Gas Chromatography with Mass Spectroscopy) type.

The method according to this invention allows the determination of the total equivalent content of hydrophobic compounds in water-miscible organic liquids in a wide range of contents and with a high sensitivity.

### Summary of drawings / figures

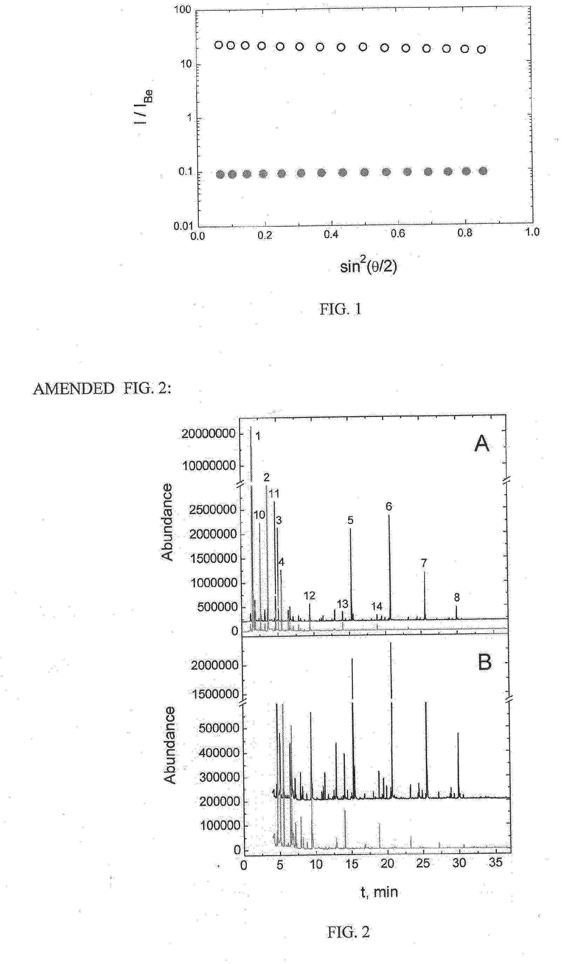

Fig. 1 shows the proof of existence of nanoparticles comprised of segregated hydrophobic compound after mixing an organic liquid with water, obtained by means of static laser light scattering method. The plot shows the angular dependence of scattering intensity from a sample of TBA (tert-butyl alcohol) mixed with water at TBA concentration c_{TBA} = 150 g/kg, both before (black empty rings) and after filtration by nanoporous filter (gray full rings), which removes the nanoparticles. The difference between intensities before and after filtration, respectively, corresponds to the scattering signal originated from the nanoparticles. All scattering intensities (including further figures) are normalized to the scattering intensity from a benzene standard I_{B}. The nanoparticles size was determined from the slope of the angular dependencies of scattered light as R= 58 nm (nanoparticles radius), D = 116 nm (nanoparticles diameter). θ is the scattering angle (an angle between the primary laser beam and the detected scattered light beam).
Fig. 2 documents the chemical composition of nanoparticles comprised of segregated hydrophobic compounds after mixing the organic liquid with water. This composition is obtained from a chemical analysis of solution before and after the nanoparticles filtering, respectively. (A) TBA (tert-butyl alcohol) mixed with water, concentration TBA c_{TBA} = 150 g/kg, before the filtration by nanoporous filter (top black curve) and after the filtration (bottom gray curve), which removed the nanoparticles. (B) A detail showing peaks with small amplitudes. Elugrams were obtained by GC-MS HS-SPME method (Gas Chromatography - Mass Spectrometry, Head Space Solid-Phase Micro Extraction). (1) TBA; (2) 2-methoxy-2-methylpropane; (3) 1-(1,1-dimethylethoxy)-2,2-dimethylpropane; (4) methylhexanol; (5) dodecane; (6) tetradecane; (7) hexadecane; (8) octadecane; (10) dimethylsilandiol; (11) hexamethylcyclotetrasiloxane; (12) octamethylcyclopentasiloxane; (13) decamethylcyclopentasiloxane; (14) dodecamethylcyclohexasiloxane. t is the elution time, Abundance is the detector signal intensity in relative units. The elugrams show that the nanoparticles are composed mostly of hydrophobic compounds: dodecane, tetradecane, hexadecane, and octadecane.

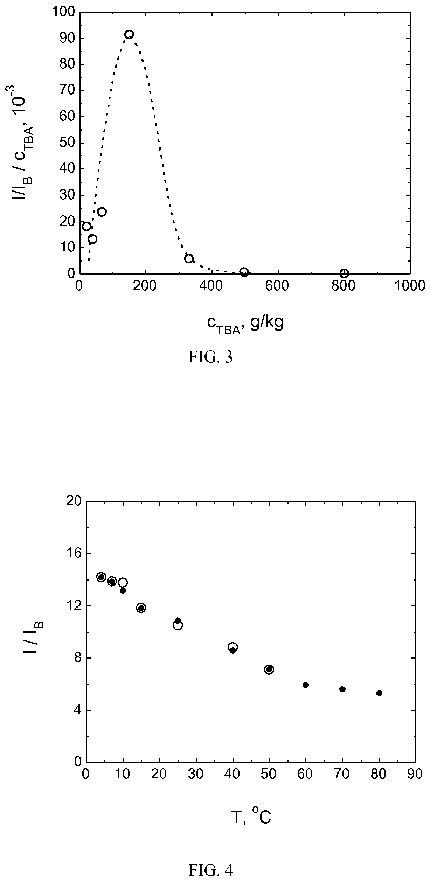

Fig. 3 shows the intensity of segregation of hydrophobic compounds as a function of the concentration of organic liquid in a mixture with water. TBA (tert-butyl alcohol) at 8 °C. The nanosegregation intensity is quantified by a benzene-normalized scattering intensity from nanoparticles I/I_{B}, normalized further by the TBA concentration c_{TBA}. The scattering angle was 45°.The nanosegregation effect can be observed within the alcohol concentration in water ranging from 1 to 60 mass %, the range practically usable for the presented method is from 3 to 45 mass %, and preferred from 10 to 20 mass %.
Fig. 4 shows the intensity of nanosegregation of hydrophobic compounds as a function of temperature. The nanosegregation intensity is quantified by the scattering intensity from the nanoparticles. Big empty rings correspond to the values measured during the mixture cooling while the small full rings during the subsequent heating of the mixture. TBA (tert-butyl alcohol) at TBA concentration c_{TBA} = 150 g/kg. The scattering angle was 45°.Temperature dependencies differ according to the particular type of organic liquid and to the particular concentration of the given organic liquid in the mixture with water.
Fig. 5 shows the example 1 of the invention embodiment.
Fig. 6 shows the example 2 of the invention embodiment.
Fig. 7 shows the example 3 of the invention embodiment.
Fig. 8 shows the example 3 of the invention embodiment.
Fig. 9 shows the example 4 of the invention embodiment.
Fig. 10 shows the example 5 of the invention embodiment.
Fig. 11 shows the example 6 of the invention embodiment.
Fig. 12 shows the example 7 of the invention embodiment.
Fig. 13 shows the example 8 of the invention embodiment.
Fig. 14 shows the example 9 of the invention embodiment.
Fig. 15 shows the example 10 of the invention embodiment.
Fig. 16 shows the example 11 of the invention embodiment.
Fig. 17 shows the example 12 of the invention embodiment.
Fig. 18 shows the example 13 of the invention embodiment.

### Examples of Invention Embodiments

### Example 1

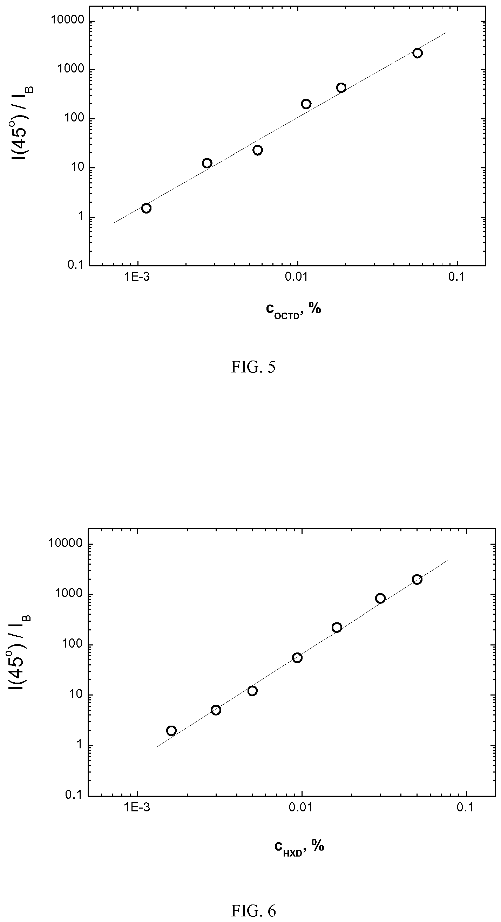

HPLC-grade (purity for high-performance liquid chromatography) ethanol was purified by distillation. Octadecane in various quantities was added to the purified ethanol so that various octadecane concentrations in ethanol were achieved. This ethanol contaminated with octadecane was then mixed with HPLC-grade water in the ratio 1:4 (20 mass % ethanol solution in water). This mixing caused generation of nanoparticles comprised of segregated octadecane. Their presence was quantified by static laser light scattering method. Fig. 5 shows the dependence of nanoparticles scattering intensity on the concentration of octadecane in ethanol.

The intensity was measured at scattering angle 45° and temperature 25 °C. The dependence in Fig. 5 is a calibration curve for the measurement of the octadecane content in ethanol, applicable for the octadecane-contaminated ethanol to water ratio 1:4 and for temperature 25 °C.

### Example 2

The procedure was the same as in the Example 1, except that hexadecane was used instead of octadecane. Fig. 6 shows the dependence of scattering intensity from the segregated hexadecane nanoparticles on hexadecane concentration in ethanol. The intensity was measured at the scattering angle 45° and temperature 25 °C. The dependence in Fig. 6 is a calibration curve for the measurement of the hexadecane content in ethanol, applicable for the hexadecane-contaminated ethanol to water ratio 1:4 and temperature 25 °C.

### Example 3

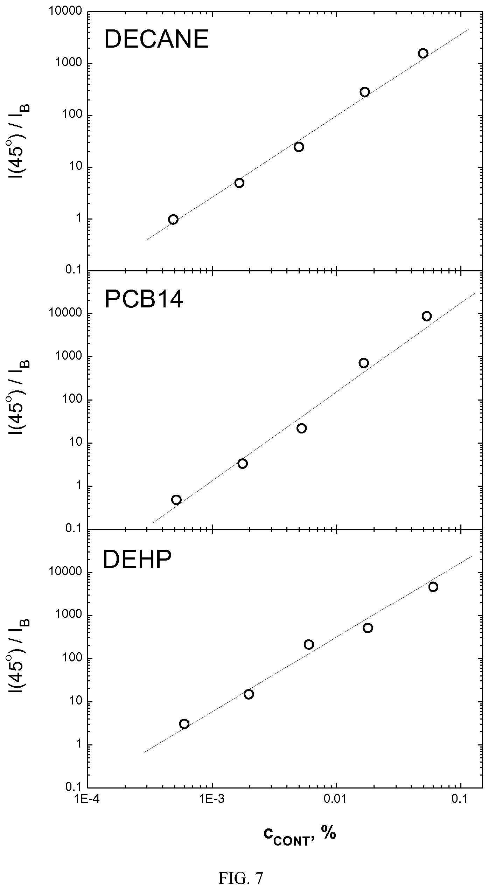

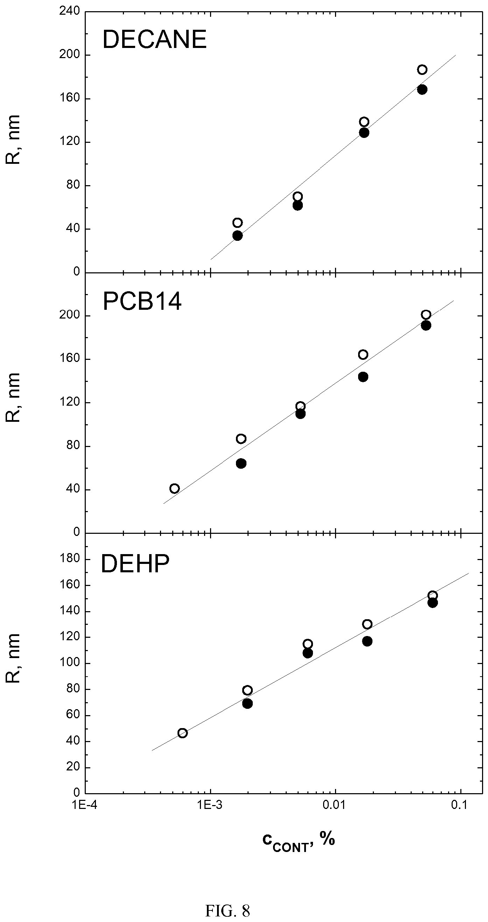

The procedure was the same as in the Example 1, except that instead of octadecane, the following hydrophobic contaminants were used: decane (Fig. 7, top), polychlorinated biphenyl 14 (Fig. 7, center), and diethylhexyl phthalate (Fig. 7, bottom). Each of the three figures shows the dependence of the scattering intensity from the nanoparticles comprised of the segregated contaminant on the concentration of corresponding contaminant in ethanol. The intensity was measured at the scattering angle 45° and temperature 25 °C. The dependencies in Fig. 7 are calibration curves for the measurement of the content of the corresponding contaminants in ethanol, applicable for the contaminated ethanol to water ratio of 1:4 and temperature 25 °C. Fig. 8 shows dependencies of the average size (radius) of the nanoparticles on the concentration of corresponding contaminant in ethanol. Radii were quantified by methods of static light scattering (empty symbols) and dynamic light scattering (full symbols).

### Example 4

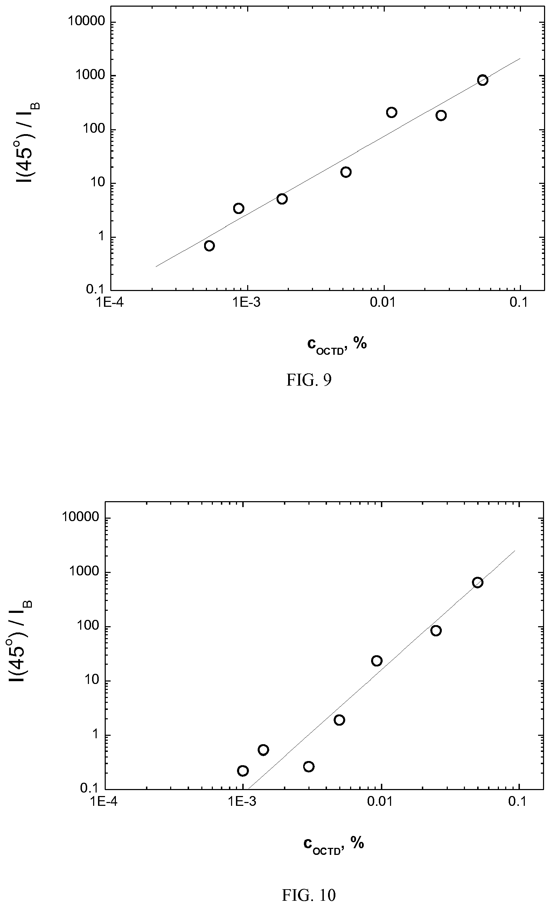

Per-analysis-grade tert-butyl alcohol (TBA) was purified by distillation. Octadecane in various quantities was added to the purified TBA so that various octadecane concentrations in TBA were achieved. This TBA contaminated with octadecane was then mixed with HPLC-grade water in the ratio 1:5.66 (15 mass % of TBA in aqueous solution). This mixing caused generation of segregated octadecane nanoparticles in solution. Their presence was quantified by laser light scattering method. Fig. 9 shows the dependence of scattering intensity from nanoparticles on the concentration of octadecane in TBA.

The intensity was measured at the scattering angle 45° and temperature 8 °C. The dependence in Fig. 9 is a calibration curve for the measurement of the octadecane content in TBA, applicable for the octadecane-contaminated TBA to water ratio 1:5.66 and temperature 8 °C.

### Example 5

The procedure was the same as in the Example 4, except that instead of TBA concentration in water of 15 mass %, the concentration of 5 mass % was used. The dependence in Fig. 10 is a calibration curve for the measurement of the octadecane content in TBA, applicable for the octadecane-contaminated TBA to water ratio 1: 19 and temperature 8 °C.

### Example 6

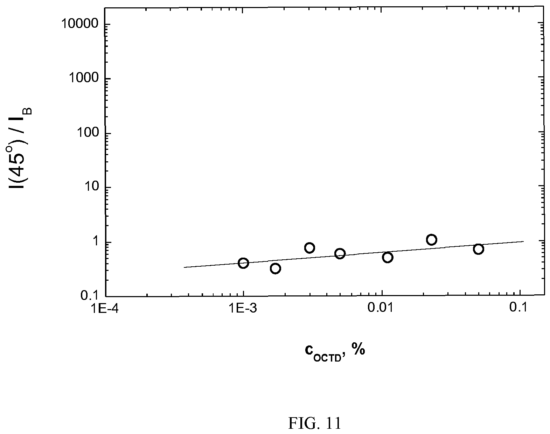

The procedure was the same as in the Example 4, except that instead of TBA concentration in water of 15 mass %, the concentration of 40 mass % was used. The dependence in Fig. 11 is a calibration curve for the measurement of the octadecane content in TBA applicable for the octadecane-contaminated TBA to water ratio 1:1.5 and temperature 8 °C.

Figures 9 to 11 illustrate the influence of concentration of the organic liquid in the mixture with water on the method efficiency. Optimum calibration curve is obtained at TBA concentration in water 15% (Fig. 9). For TBA concentrations close to the concentrations limiting with respect to the applicability of this method (from 5% to 40%), it is apparent that for 5%TBA concentration the calibration curve is less accurate, with greater scatter mainly for the lower contents of octadecane under 0.01% (Fig. 10); and for 40% TBA concentration the calibration curve is already relatively flat, which means lower sensitivity of the method and lower precision of the determination of the content of hydrophobic compounds(Fig. 11).

### Example 7

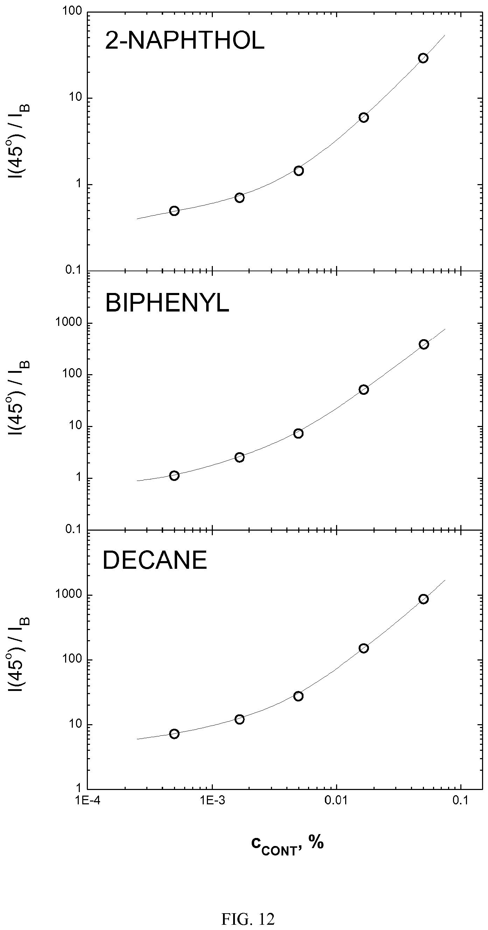

The procedure was the same as in the Example 4, except that instead of octadecane, the following hydrophobic contaminants were used: 2-naphtol (Fig. 12, top), biphenyl 14 (Fig. 12, center), and decane (Fig. 12, bottom). Each of the three figures shows the dependence of scattering intensity from nanoparticles of the segregated contaminant on the concentration of the corresponding contaminant in TBA. The scattering angle was 45° and temperature was 25 °C. The dependencies in Fig. 12 are calibration curves for the measurement of the content of the corresponding contaminants in TBA applicable for the contaminated TBA to water ratio of 1:5.66 and temperature 25 °C.

### Example 8

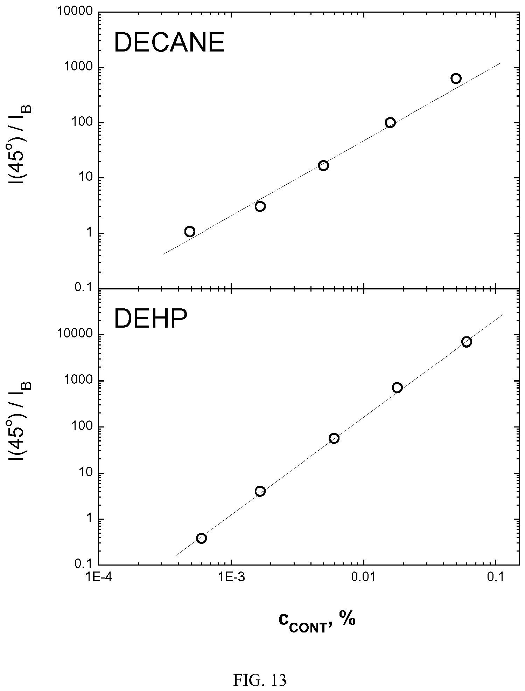

The procedure was the same as in the Example 1, except that isopropyl alcohol was used instead of ethanol, and the following hydrophobic contaminants were used: decane (Fig. 13, top) and diethylhexyl phthalate (Fig. 13, bottom). Both figures show the dependence of scattering intensity from nanoparticles of the segregated contaminant on the concentration of the corresponding contaminant in isopropyl alcohol. The scattering angle was 45° at temperature 25 °C. The dependencies in Fig. 13 are calibration curves for the measurement of the content of the corresponding contaminants in isopropyl alcohol applicable for the contaminated isopropyl alcohol to water ratio 1:4 and temperature 25 °C.

### Example 9

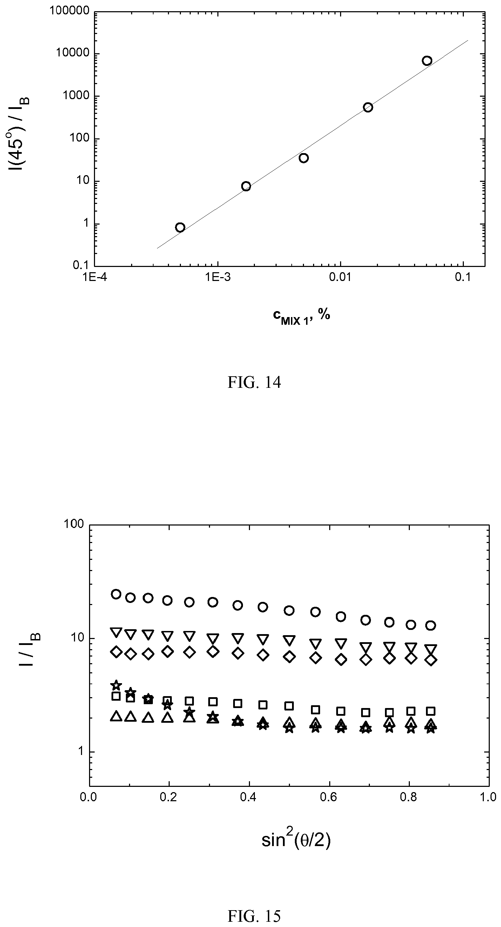

The procedure was the same as in the Example 1. A 1:1:1 mixture of the following hydrophobic compounds was used as the contaminant: decane, polychlorinated biphenyl 14 (PCB14) and benzo[a]pyrene. Fig. 14 shows the dependence of scattering intensity from the nanoparticles of segregated contaminant on the overall contaminant concentration c_{MIX1}in ethanol. The scattering angle was 45° and temperature was 25°C. Table 1 shows values of intensity of scattering from nanoparticles comprised of segregated contaminants on the concentration of individual contaminants in ethanol (for the cases where only one type of contaminant is present in ethanol) and for various concentrations of the above-defined mixture of contaminants in ethanol. The dependence in Fig. 14 is a calibration curve for the measurement of the content of the 1:1:1 mixture decane/PCB14/benzo[a]pyrene in ethanol, applicable for the ethanol contaminated by this mixture to water ratio 1:4 and temperature 25 °C.

**Table 1**

| C_{cont}, % | I(45°) / I_{B} | | | | | | |
|---|---|---|---|---|---|---|---|
| | decane | octadecane | benzo[a]pyrene | PCB14 | DEHP | MIXTURE 1 | MIXTU RE 2 |
| 0.05000 | 1559.49 | 2148.09 | 5125.59 | 8670.06 | 4612.03 | 6740.23 | |
| 0.01670 | 280.61 | 425.33 | 1273.75 | 698.35 | 501.26 | 545.25 | |
| 0.00500 | 24.53 | 22.68 | 84.71 | 21.8 | 207.73 | 34.88 | 64.34 |
| 0.00167 | 4.92 | 6.23 | 18.63 | 3.31 | 14.74 | 7.54 | |
| 0.00100 | 3.01 | 2.05 | 10.53 | 1.86 | 7.37 | | |
| 0.00050 | 0.97 | 0.71 | 1.50 | 0.48 | 3.01 | 0.82 | |

### Example 10

The procedure was the same as in the Example 1. A mixture of the following hydrophobic compounds was used as the contaminant: decane, octadecane, PCB14, diethylhexyl phthalate and benzo[a]pyrene in the mutual ratio 1:1:1:1:1. Fig. 15 shows angular dependencies of scattering intensity from particles of segregated contaminant in ethanol: for the 1:1:1:1:1 mixture decane/octadecane/PCB14/diethylhexyl phthalate/benzo[a]pyrene (with overall total concentration of the contaminants in ethanol 0.005% - black rings); for just one contaminant in ethanol - decane (concentration 0.001% - black squares); for just one contaminant in ethanol - octadecane (concentration 0.001% - black triangles pointed upwards); for just one contaminant in ethanol - PCB14 (concentration 0.001% - black stars); for just one contaminant in ethanol - diethylhexyl phthalate (concentration 0.001% - black rhombs); for just one contaminant in ethanol - benzo[a]pyrene (concentration 0.001% - black triangles pointed downwards). The scattering angle was 45° and temperature was 25 °C. Table 1 shows values of intensity of scattering from nanoparticles comprised of segregated contaminants for various concentration of individual contaminants in ethanol (for the cases where only one type of contaminant is present in ethanol) and for the contaminants mixture defined in Example 10 (mixture 2). Example 10 illustrates the so-called additivity, i.e., the fact that if more types of contaminants are present in the organic liquid, the scattering signals are adding up.

### Example 11

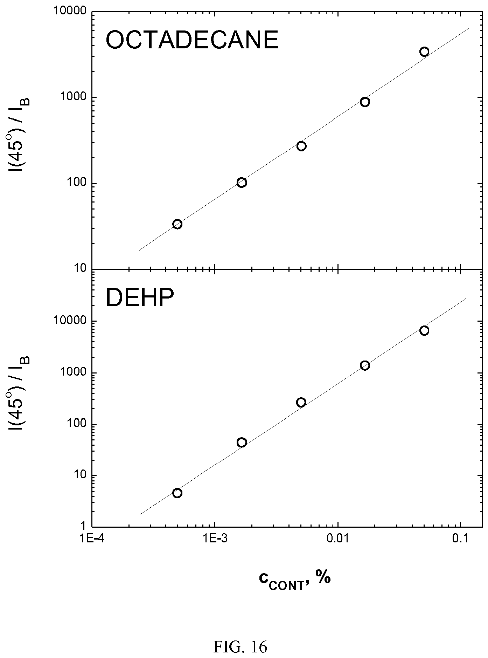

The procedure was the same as in the Example 1, except that acetone was used instead of ethanol, and the following hydrophobic contaminants were used: octadecane (Fig. 16, top) and diethylhexyl phthalate (Fig. 16, bottom). Both figures show the dependence of scattering intensity from nanoparticles of segregated contaminants on concentration of the corresponding contaminant in acetone. The scattering angle was 45° and temperature was 25°C.The dependencies in Fig. 16 are calibration curves for the measurement of the content of corresponding contaminants in acetone applicable for the contaminated acetone to water ratio 1:4 and temperature 25 °C.

### Example 12

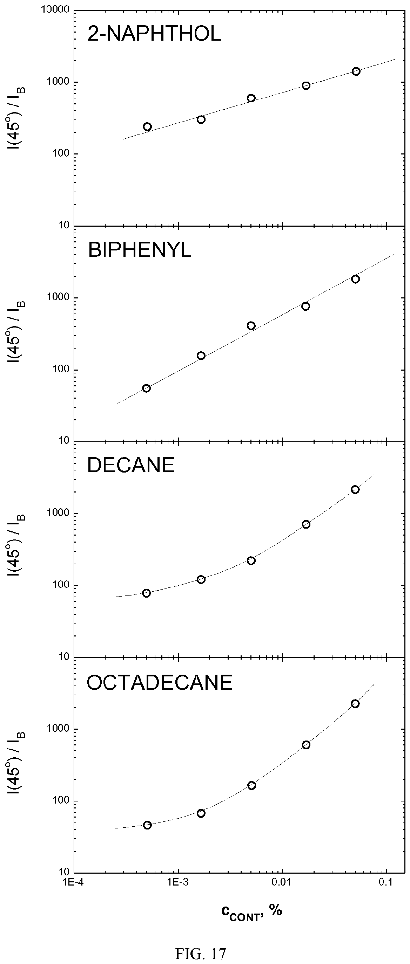

The procedure was the same as in the Example 1, except that dimethoxyethane was used instead of ethanol, and the following hydrophobic contaminants were used (Fig. 17 downwards): 2-naphtol, biphenyl, decane, and octadecane. Each of the four plots in Fig. 17 show dependence of scattering intensity from nanoparticles comprised of the segregated contaminant on concentration of the corresponding contaminant in dimethoxyethane. The scattering angle was 45° and temperature was 25°C. The dependencies in Fig. 17 are calibration curves for the measurement of the content of corresponding contaminants in dimethoxyethane applicable for the contaminated dimethoxyethane to water ratio 1:5.66 and measurement temperature 25 °C.

### Example 13

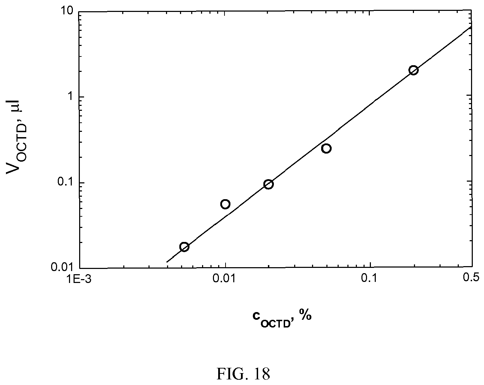

The procedure was the same as in the Example 1. The presence of nanoparticles comprising of segregated octadecane was quantified by nanoparticle tracking analysis method. Fig. 18 shows the dependence of the total volume of all nanoparticles in 1 mililiter of the mixture on the concentration of octadecane in ethanol. The dependence in Fig. 18 is a calibration curve for the measurement of the octadecane content in ethanol, applicable for the octadecane-contaminated ethanol to water ratio 1:4 and for temperature 25 °C.

The legal protection of the method for determination of the content of hydrophobic compounds in water-miscible organic liquids by the presented invention is not limited to the use of either static or dynamic laser light scattering or nanoparticle tracking analysis method; also other methods of quantitative analysis can be used.

The determination of content of hydrophobic compounds in contaminated organic liquids by means of calibration curves has to be carried out under conditions (organic liquid to water ratio in the mixture and temperature) identical to those applied during acquiring calibration curves.

As shown in the examples, the method presented herein is applicable for contaminant concentrations from 5 to 10³ ppm, whereas this range can be preferably extended by sample pretreatment (e.g. by 100-times diluting or concentrating) to 50ppb - 10⁵ ppm.

### Industrial applicability

The method of hydrophobic compounds determination in organic liquids is important mainly because of the need for use of pure organic liquids (mainly alcohols) in food industry, medicine, and chemical industry.

## Claims

1. A method for determination of the content of hydrophobic compounds in water-miscible organic liquids using static laser light scattering or dynamic laser light scattering based on the measurement of the nanoparticles average size, **characterized in that** organic liquid containing hydrophobic compounds is mixed with water, where the organic liquid content in such a mixture ranges from 3 to 45 mass % and the content of hydrophobic compounds in water-miscible organic liquid ranges from 0.0005% to 0.1 mass %, at temperatures at which such a mixture is in a liquid phase, hydrophobic compounds contained in the organic liquid segregate into nanoparticles whose concentration, size and density correlate with the content of hydrophobic compounds in the organic liquid and subsequently the content of hydrophobic compounds in the organic liquid is quantified by means of calibration dependencies of scattering intensity or nanoparticle size for the content of hydrophobic compounds in the organic liquid, where calibration curves are obtained for the range of contents of hydrophobic compounds in the organic liquid up to 0.1 mass %; and each calibration curve is acquired for a selected value of the organic liquid content in the mixture with water within the range from 3 to 45 mass %, and at the same temperature as measurements of samples with unknown content of hydrophobic compounds.

2. A method according to claim 1 wherein hydrophobic compounds are alkanes, polychlorinated biphenyls, polycyclic aromatic hydrocarbons, and phthalates.

3. A method according to claims 1 and 2 wherein the alkanes are decane, dodecane, tetradecane, hexadecane, and octadecane.

4. A method according to claims 1 and 2 wherein the polychlorinated biphenyls are polychlorinated biphenyl 14.

5. A method according to claims 1 and 2 wherein the polycyclic aromatic hydrocarbons are benzo[a]pyrene, naphtol, and biphenyl.

6. A method according to claims 1 and 2 wherein the phthalates are diethylhexyl phthalate.

7. A method according to claim 1 wherein the organic liquid miscible with water is alcohol, acetone, and dimethoxyethane.

8. A method according to claims 1 and 7 wherein the alcohols are ethanol, isopropyl alcohol, and tert-butyl alcohol.

9. A method according to claims from 1 to 8 wherein the content of the organic liquid containing hydrophobic compounds in the mixture with water is from 5 to 25 mass %.

10. A method according to claims from 1 to 8 wherein the content of the organic liquid containing hydrophobic compounds in the mixture with water is from 10 to 20 mass %.

## Patentansprüche

1. Verfahren zur Bestimmung des Gehalts an hydrophoben Verbindungen in wassermischbaren organischen Flüssigkeiten mittels statischer Laserlichtstreuung oder dynamischer Laserlichtstreuung basierend auf der Messung der durchschnittlichen Größe von Nanopartikeln, **dadurch gekennzeichnet, dass** die organische Flüssigkeit, die hydrophobe Verbindungen enthält, mit Wasser gemischt wird, wobei der Gehalt an organischer Flüssigkeit in einer solchen Mischung im Bereich von 3 bis 45 Massen-% liegt und der Gehalt an hydrophoben Verbindungen in der wassermischbaren organischen Flüssigkeit im Bereich von 0,0005 % bis 0,1 Massen-% liegt, bei Temperaturen, bei denen sich eine solche Mischung in flüssiger Phase befindet, hydrophobe Verbindungen, die in der organischen Flüssigkeit enthalten sind, segregieren in Nanopartikel, deren Konzentration, Größe und Dichte mit dem Gehalt an hydrophoben Verbindungen in der organischen Flüssigkeit korrelieren, und anschließend wird der Gehalt an hydrophoben Verbindungen in der organischen Flüssigkeit quantifiziert mittels Kalibrierungsabhängigkeiten von Streuintensität oder Größe von Nanopartikeln für den Gehalt an hydrophoben Verbindungen in der organischen Flüssigkeit, wo Kalibrierkurven für den Bereich der Gehalte an hydrophoben Verbindungen in der organischen Flüssigkeit bis 0,1 Massen-% erhalten werden; und jede Kalibrierkurve wird für einen ausgewählten Wert des Gehalts an organischer Flüssigkeit in der Mischung mit Wasser im Bereich von 3 bis 45 Massen-% erhalten, und bei der gleichen Temperatur wie Messungen von Proben mit unbekanntem Gehalt an hydrophoben Verbindungen.

2. Verfahren nach Anspruch 1, wobei die hydrophoben Verbindungen Alkane, polychlorierte Biphenyle, polycyclische aromatische Kohlenwasserstoffe und Phthalate sind.

3. Verfahren nach den Ansprüchen 1 und 2, wobei die Alkane Decan, Dodecan, Tetradecan, Hexadecan und Octadecan sind.

4. Verfahren nach den Ansprüchen 1 und 2, wobei die polychlorierten Biphenyle polychloriertes Biphenyl 14 sind.

5. Verfahren nach den Ansprüchen 1 und 2, wobei die polycyclischen aromatischen Kohlenwasserstoffe Benzo[a]pyren, Naphtol und Biphenyl sind.

6. Verfahren nach den Ansprüchen 1 und 2, wobei die Phthalate Diethylhexylphthalat sind.

7. Verfahren nach Anspruch 1, wobei die mit Wasser mischbare organische Flüssigkeit Alkohol, Aceton und Dimethoxyethan ist.

8. Verfahren nach den Ansprüchen 1 und 7, wobei die Alkohole Ethanol, Isopropylalkohol und tert-Butylalkohol sind.

9. Verfahren nach den Ansprüchen 1 bis 8, wobei der Gehalt an organischer Flüssigkeit, die hydrophobe Verbindungen enthält, in der Mischung mit Wasser 5 bis 25 Massen-% beträgt.

10. Verfahren nach den Ansprüchen 1 bis 8, wobei der Gehalt an organischer Flüssigkeit, die hydrophobe Verbindungen enthält, in der Mischung mit Wasser 10 bis 20 Massen-% beträgt.

## Revendications

1. Une méthode pour déterminer le contenu de composants hydrophobes dans les liquides organiques miscibles à l'eau, à travers la diffusion statique de la lumière et la diffusion dynamique de la lumière, sur la base de la mesure de la taille moyenne des nanoparticules, **caractérisé en ce que** le liquide organique contenant les composés hydrophobes est mélangé avec de l'eau, où le contenu de liquide organique dans ledit mélange oscille entre 3 et 45 %ₘₐₛ et le contenu de composés hydrophobes dans le liquide organique miscible à l'eau oscille entre 0,0005 et 0,1 %ₘₐₛ - à des températures auxquelles ledit mélange est dans une phase liquide, les composés hydrophobes contenus dans le liquide organique se séparent en nanoparticules, dont la concentration, la taille et la densité sont corrélées avec le contenu de composés hydrophobes dans le liquide organique et, par la suite, le contenu de composés hydrophobes dans le liquide organique est quantifié à l'aide des dépendances d'étalonnage de l'intensité de la diffusion ou de la taille des nanoparticules pour le contenu des composés hydrophobes dans le liquide organique, où les courbes d'étalonnage sont obtenues pour la gamme du contenu de composés hydrophobes dans le liquide organique jusqu'à 0,1 %ₘₐₛ, et chaque courbe d'étalonnage est acquise pour une valeur sélectionnée du contenu de liquide organique dans le mélange avec l'eau dans une fourchette comprise entre 3 et 45 %ₘₐₛ, et à la même température comme mesures d'échantillons avec un contenu inconnu de composés hydrophobes.

2. Une méthode selon la revendication 1 où les composés hydrophobes sont des alcanes, biphényles polychlorés, hydrocarbures polycycliques aromatiques et phtalates.

3. Une méthode selon les revendications 1 et 2 où les alcanes sont décane, dodécane, tétradécane, hexadécane et octadécane.

4. Une méthode selon les revendications 1 et 2 où les biphényles polychlorés sont biphényle polychloré 14.

5. Une méthode selon les revendications 1 et 2 où les hydrocarbures polycycliques aromatiques sont benzo[a]pyrène, naphtol et biphényle.

6. Une méthode selon les revendications 1 et 2 où les phtalates sont phtalate de diéthylhexyle.

7. Une méthode selon la revendication 1 où le liquide organique miscible à l'eau est alcool, acétone et diméthoxyethane.

8. Une méthode selon les revendications 1 et 7 où les alcools sont éthanol, alcool isopropylique et alcool tert-butylique.

9. Une méthode selon les revendications 1 et 8 où le contenu du liquide organique contenant les composés hydrophobes dans ledit mélange avec l'eau oscille entre 5 et 25 %ₘₐₛ.

10. Une méthode selon les revendications 1 et 8 où le contenu du liquide organique contenant les composés hydrophobes dans ledit mélange avec l'eau oscille entre 10 et 20 %ₘₐₛ.
